(19) Europäisches Patentamt
European Patent Office
Office européen des brevets

(11) **EP 4 245 827 A1**

(12) **EUROPÄISCHE PATENTANMELDUNG**

(43) Veröffentlichungstag:
**20.09.2023   Patentblatt 2023/38**

(21) Anmeldenummer: **23161186.4**

(22) Anmeldetag: **10.03.2023**

(51) Internationale Patentklassifikation (IPC):
***C09K 11/77*** $^{(2006.01)}$

(52) Gemeinsame Patentklassifikation (CPC):
**C09K 11/77062; C09K 11/77742;** A61L 2101/02

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **17.03.2022   EP 22162615**

(71) Anmelder: **Evonik Operations GmbH**
**45128 Essen (DE)**

(72) Erfinder:
• **Schulte, Simone**
  **45133 Essen (DE)**
• **Huth, Michael**
  **63477 Maintal (DE)**
• **Fischer, Stefan**
  **59494 Soest (DE)**
• **Janke, Christina**
  **45327 Essen (DE)**
• **Tschernjaew, Juri**
  **63743 Aschaffenburg (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(54)   **VERFAHREN ZUR HERSTELLUNG VON GEFLUXTEN UP-CONVERSION LEUCHTSTOFFEN**

(57)   Die Erfindung betrifft Verfahren zur Herstellung eines Up-Conversion Leuchtstoffes der allgemeinen Formel (I)

$$A_{1-x-y-z}B^*_yB_2SiO_4{:}Ln^1_x,Ln^2_z, \qquad I$$

umfassend folgende Schritte:
- i) Bereitstellung mindestens eines Lanthanoidsalzes,
- ii) Bereitstellung eines Silikats oder eines Siliziumdioxids,
- iii) Bereitstellung mindestens eines Erdalkalimetallsalzes und mindestens eines Alkalimetallsalzes,
- iv) Bereitstellung mindestens eines Flussmittels,
- a) Mischen der Komponente i), ii) iii) und iv) mittels Mahlens zur Erhaltung einer Mischung, oder
- b) Mischen der Komponente i), ii) und iii) und iv) in einem organischen polaren oder unpolaren Lösemittel, das kein protisches Lösemittel ist, mittels Mahlens zur Erhaltung einer Mischung;
- c) Vorkalzinierung der Mischung
- d) Kalzinierung der Mischung
- e) Erhalten eines silikatbasierten Up-Conversion Leuchtstoffes der allgemeinen Formel (I), bevorzugt nach Abkühlen des Materials,
wobei mindestens 3,5 Gew.-% Flussmittel, bezogen auf die Gesamtmenge der Edukte, eingesetzt werden.

EP 4 245 827 A1

**Beschreibung**

Die Erfindung betrifft ein Verfahren zur Herstellung von gefluxten Up-Conversion Leuchtstoffen, den gefluxten Up-Conversion Leuchtstoff und dessen Verwendung in Beschichtungen mit anti-mikrobieller Eigenschaft.

Tagtäglich sind Menschen Millionen von Mikroorganismen wie Bakterien, Pilzen und Viren ausgesetzt. Viele dieser Mikroorganismen sind nützlich bzw. sogar notwendig. Dennoch gibt es neben den harmloseren Vertretern auch krankheitsverursachende oder sogar tödliche Bakterien, Pilze und Viren.

Durch den täglichen Umgang mit anderen Menschen und den Kontakt mit Gegenständen, die andere benutzt haben, können Mikroorganismen übertragen werden. Die antimikrobielle Ausstattung von Oberflächen erfolgt insbesondere in hygienesensiblen Bereichen. Anwendungsbereiche sind vor allem Oberflächen von medizintechnischen Geräten und Bedarfsgegenstände in Krankenhäusern sowie in Einrichtungen des ambulanten Gesundheits- und Sozialwesens. Hinzu kommen Oberflächen im öffentlichen Raum, im Lebensmittelsektor und in der Tierhaltung. Die Verbreitung von pathogenen Mikroorganismen stellt heute ein großes Problem im Pflegebereich und in der Medizin dar, und auch überall dort, wo viele Menschen auf engem Raum verkehren. Ein besonders Risiko stellt gegenwärtig auch das vermehrte Aufkommen sogenannter multiresistenter Keime dar, die unempfindlich gegen übliche Antibiotika geworden sind.

Um das Risiko der Verbreitung von pathogenen Erregern über Berührungsoberflächen zu verringern, werden, neben Maßnahmen zur Standardhygiene, antimikrobielle Technologien und Werkstoffe genutzt. Chemische Substanzen oder die Verwendung physikalischer Verfahren können den Vermehrungsprozess von Mikroorganismen kritisch beeinflussen. Zu den physikalischen Methoden zählen z. B. Hitze, Kälte, Strahlung oder Ultraschall, etc. Bei den chemischen Methoden sind Halogene, Metallionen, organische Verbindungen und Farbstoffe, giftige Gase, etc. bekannt.

Obwohl in den meisten Fällen chemische und physikalische Methoden außerordentlich effektiv bei der Zerstörung von Mikroorganismen sind, so haben sie doch nur einen kurzzeitigen Effekt, chemische Methoden fördern die Entstehung von Resistenzen und sind unter Umständen für manche Anwendungen nicht geeignet, da sie zur Zerstörung der zu schützenden Oberflächen führen. Den größten Nachteil stellt allerdings, gerade bei chemischen, organischen Substanzen, die Gefährlichkeit bzw. Giftigkeit für den Menschen dar. Bestimmte Substanzen, wie z. B. Formaldehyd, welches viele Jahre als Desinfektionsmittel Anwendung fand, stehen inzwischen im Verdacht, Krebs zu verursachen oder extrem umweltschädlich zu sein.

Oberflächen mit antimikrobieller Wirkung könnten einen entscheidenden Beitrag zur Lösung dieser Probleme leisten. Die heute gängigen Verfahren zur Erzeugung solcher antimikrobiellen Eigenschaften verwenden überwiegend in das Material eingearbeitete Wirkstoffe wie z.B. Silberpartikel, Kupferpartikel, deren Metalloxide oder quaternäre Ammoniumverbindungen. Dabei werden die antimikrobiell wirkenden Metalle, Metalloxide oder Metalloxidgemische häufig zu Nanoteilchen verarbeitet und dann Farben, Lacken oder Polymerwerkstoffen zugemischt. Der breite Einsatz von Metallpartikeln ist in Frage zu stellen, da die langfristige Wirkung dieses Schwermetalls auf Menschen und Umwelt kaum abzuschätzen ist.

Beispielsweise offenbart die WO 2019/197076 Partikel, die mit einer Schicht ausgestattet sind, die sowohl Antimon-Zinnoxid als auch Manganoxid enthält. Dem Fachmann ist bekannt, dass die antimikrobiellen Oberflächen aufgrund des elektrochemischen Verhaltens von Metallen erzeugt werden, die bei Anwesenheit von Feuchtigkeit mikrogalvanische Zellen und durch die mikroelektrischen Felder keimabtötende Wirkung entfalten.

Es ist ebenso bekannt, UV-Strahlung in der Medizin oder in der Hygiene einzusetzen, um beispielsweise Wasser, Gase oder Oberflächen zu desinfizieren. So wird in der Trinkwasseraufbereitung seit langem UV-Strahlung zur Reduzierung der Anzahl von fakultativ krankheitserregenden Mikroorganismen im Wasser eingesetzt. Dabei wird vorzugsweise UV-C-Strahlung im Wellenlängenbereich zwischen 200 nm und 280 nm eingesetzt. Der Einsatz elektromagnetischer Strahlung mit unterschiedlichen Wellenlängen sollte die unterschiedliche Absorption der verschiedenen Proteine, den in Mikroorganismen, Geweben oder Zellen enthaltenden Amino-/Nukleinsäuren (z.B. DNA oder RNA) sowie Peptidbindungen zwischen den einzelnen Säuren berücksichtigen. So absorbiert DNA/RNA elektromagnetische Strahlung innerhalb des Wellenlängenbereichs zwischen 200 nm und 300 nm gut und zwischen 250 nm und 280 nm besonders gut, so dass diese Strahlung zur Inaktivierung von DNA/RNA besonders geeignet ist. Es können also krankheitserregende Mikroorganismen (Viren, Bakterien, Hefen, Schimmelpilze u.a.) mit einer solchen Bestrahlung inaktiviert werden. Je nach Dauer und Intensität der Bestrahlung kann die Struktur von DNA bzw. RNA zerstört werden. Somit werden stoffwechselaktive Zellen inaktiviert und/oder deren Vermehrungsvermögen kann beseitigt werden. Vorteilhaft bei der Bestrahlung mit UV-Strahlung ist, dass die Mikroorganismen keine Resistenz dagegen entwickeln können. Diese physikalischen Methoden erfordern allerdings spezielle Apparaturen und müssen in der Regel von geschultem Personal regelmäßig wiederholt werden, was einen breiten Einsatz dieser Methoden erschwert.

Des Weiteren ist es neben einer direkten Bestrahlung mit elektromagnetischer Strahlung aus dem Wellenlängenbereich der UV-Strahlung auch bekannt, den Effekt der so genannten Up-Conversion auszunutzen. Dabei werden Leuchtstoffpartikel eingesetzt, mit denen elektromagnetische Strahlung mit Wellenlängen oberhalb des UV-Strahlung, insbesondere sichtbares Licht oder Infrarotstrahlung, in elektromagnetische Strahlung mit geringerer Wellenlänge umgewandelt werden kann, so dass die Emission von in gewünschter Wellenlänge wirkender Strahlung von den einzelnen Leuchtstoffpartikeln erreicht werden kann.

DE 10 2015 102 427 betrifft einen elektromagnetische Strahlung im Wellenlängenbereich des UV-Lichts emittierenden Körper. Bei dem Körper sind in einem oberflächennahen Bereich innerhalb des Werkstoffs, aus dem der Körper gebildet ist oder einer Beschichtung auf dem Körper, Leuchtstoffpartikel eingebettet. Hierbei wird nur allgemein angegeben, dass die Leuchtstoffpartikel einer auf dem Werkstoff auszubildenden Beschichtung direkt bei der Verarbeitung zugegeben werden, dabei sollte der jeweilige Werkstoff eine geeignete Konsistenz bzw. Viskosität aufweisen. Hinsichtlich geeigneter Polymere und Additive schweigt DE 10 2015 102 427.

US 2009/0130169 A1 bzw. WO 2009/064845 A2 beschreibt Leuchtstoffe, die in Polyvinylchloride, Acrylbutadiene, Polyolefine, Polycarbonate, Polystyrole oder Nylon eingebracht werden können, welche durch die Up-Conversion Eigenschaft der Leuchtstoffe pathogene Mikroorganismen abtöten. Es handelt sich hierbei um Leuchtstoffe, die bei einer Temperatur von 1800 - 2900 °C hergestellt werden. Die US 2009/0130169 A1 bzw. WO 2009/064845 A2 offenbart zwar eine Zusammensetzung enthaltend besagter Leuchtstoffe mit behaupteter antimikrobieller Wirkung, zeigt jedoch weder einen Nachweis der Up-Conversion Eigenschaft noch mikrobiologischer Untersuchungen. Das darin offenbarte Verfahren führt zu keinem Leuchtstoff, der eine Up-Conversion Eigenschaft aufweist, sondern zu einem amorphen und glasartigen Produkt.

Darüber hinaus schweigt die US 2009/0130169 A1 bzw. WO 2009/064845 A2 über die Verträglichkeit der Komponente in der Beschichtungszusammensetzung oder die Eigenschaften der Beschichtungsoberflächen, wie etwa die Lackoberflächen. Das Erscheinungsbild von Beschichtungsoberflächen spielen jedoch beim Anwender eine besondere Rolle.

Die Anforderungen an Lacke und Farben sind vielfältig. Grundsätzlich haben Lack- bzw. Farbbeschichtungen zwei Aufgaben bzw. Funktionen, die schützende und die dekorative Funktion. Sollten im Weiteren nur der Begriff "Lackbeschichtung" aufgeführt sein, so sind beide Arten der Beschichtung gemeint. Sie verschönern, schützen und bewahren Materialien wie Holz, Metall oder Kunststoff. Demnach werden auf der einen Seite brillante und glatte Lackschichten gefordert, auf der anderen Seite eine geschlossene Lackschicht zur Sicherstellung der chemischen und mechanischen Beständigkeit, eine gewisse Gleitfähigkeit der Lacke oder eine besondere Haptik.

Im Gegensatz zu der WO 2009/064845 A2 konnte die Patentanmeldung PCT/EP2020/077798 Leuchtstoffe und deren Herstellung offenbaren, die eine Up-Conversion aufweisen. Solche Leuchtstoffe können bei Bestrahlung mit elektromagnetischer Strahlung mit geringerer Energie und höherer Wellenlänge im Bereich von 2000 nm bis 400 nm, insbesondere im Bereich von 800 nm bis 400 nm, elektromagnetische Strahlung mit höherer Energie bzw. kürzerer Wellenlänge im Bereich von 400 nm bis 100 nm, bevorzugt im Bereich von 300 nm bis 200 nm emittieren, so dass sie geeignet sind, als anti-mikrobielle Leuchtstoffe in Lackbeschichtungen eingesetzt zu werden.

So beschreibt die EP 3929254 eine Zusammensetzung enthalten mindestens ein filmbildendes Polymer, mindestens einen Up-Conversion Leuchtstoff gemäß der Lehre der PCT/EP2020/077798, optional mindestens ein Additiv und optional mindestens einen Härter. Es konnte gezeigt werden, dass Lackbeschichtungen enthaltend dieser Leuchtstoffe antimikrobiell wirken und ohne dass die übrigen Eigenschaften, insbesondere die Lagerstabilität, signifikant beeinträchtigt werden.

Es wurde jedoch auch festgestellt, dass die Leuchtstoffe, hergestellt nach einem Verfahren gemäß PCT/EP2020/077798, eine inhomogene Partikelgrößenverteilung aufweisen, was bei deren Einarbeitung in einer Lackmatrix eine besondere Herausforderung darstellt. Auch wenn die Lehre der EP 3929254 zu anti-mikrobiellen wirkenden Lackbeschichtungen führt, so wäre es wünschenswert, wenn die Intensität der Emission der Leuchtstoffe erhöht werden könnte.

Aus der noch nicht offengelegten Europäischen Patentanmeldung EP 21167984.0 wurde vorgeschlagen, dass man zur Herstellung von Beschichtungen mit anti-mikrobieller Eigenschaft enthaltend

- mindestens ein filmbildendes Polymer,
- optional mindestens ein Additiv,
- optional mindestens einen Härter,
- mindestens einen Up-Conversion Leuchtstoff der allgemeinen Formel (I)

$$A_{1-x-y-z}B^*_yB_2SiO_4:Ln^1_x,Ln^2_z, \qquad I$$

mit

x = 0,0001 - 0,0500;
z = 0,0000 oder z = 0,0001 bis 0,3000 mit der Maßgabe, dass gilt: y = x + z;
A ausgewählt aus der Gruppe bestehend aus Mg, Ca, Sr und Ba;
B ausgewählt aus der Gruppe bestehend aus Li, Na, K, Rb und Cs;

B* ausgewählt aus der Gruppe bestehend aus Li, Na und K, wobei B gleich B* oder B ungleich B* ist, bevorzugt B und B* sind ungleich;

$Ln^1$ ausgewählt aus der Gruppe bestehend aus Praseodym (Pr), Erbium (Er) und Neodym (Nd);

$Ln^2$ ausgewählt aus Gadolinium (Gd),

einen Leuchtstoff, der mit mindestens einem halogenhaltigen Flussmittel hergestellt wurde, verwendet. Hierbei werden höchstens 3,5 Gew.-% Flussmittel, bezogen auf die Gesamtmenge der Edukte, eingesetzt.

Ausgehend von der noch nicht offengelegten Europäischen Patentanmeldung EP 21167984.0 wäre es daher wünschenswert, wenn der Leuchtstoff verbessert wird und zudem das Verfahren zu dessen Herstellung optimiert werden könnte.

Aus dem Stand der Technik sind zahlreiche Flussmittel aller Art, wie Halogenide, Carbonate, Sulfate, Oxide und Borate von jeweils, wo zutreffend, Ammonium, Lithium, Natrium, Kalium, Rubidium, Cäsium, Magnesium, Calcium, Strontium, Barium, Blei, Lanthan, Lutetium, Aluminium, Bismut sowie Borsäure, dem Fachmann bekannt. Auch bekannt sind deren Einsatzgebiete im Bereich der Metallurgie, um beispielsweise das Kristallwachstum zu beschleunigen oder die Bildung von Fremdphasen zu unterbinden.

Flussmittel wird im englischsprachigen Raum als "flux" übersetzt; daher ist auch der Begriff Fluxmittel bekannt. Die Behandlung mit Flussmittel wird auch fluxen genannt bzw. das Produkt wurde gefluxt.

Überraschend konnte die Aufgabe durch ein Verfahren gemäß des Hauptanspruchs gelöst werden.

Hierbei wird ein Verfahren zur Herstellung eines Up-Conversion Leuchtstoffes der allgemeinen Formel (I)

$$A_{1-x-y-z}B^*{}_yB_2SiO_4{:}Ln^1{}_x,Ln^2{}_z, \qquad I$$

mit

$x = 0,0001 - 0,0500$;

$z = 0,0000$ oder $z = 0,0001$ bis $0,3000$ mit der Maßgabe, dass gilt: $y = x + z$;

A ausgewählt aus der Gruppe bestehend aus Mg, Ca, Sr und Ba;

B ausgewählt aus der Gruppe bestehend aus Li, Na, K, Rb und Cs;

B* ausgewählt aus der Gruppe bestehend aus Li, Na und K, wobei B gleich B* oder B ungleich B* ist, bevorzugt B und B* sind ungleich;

$Ln^1$ ausgewählt aus der Gruppe bestehend aus Praseodym (Pr), Erbium (Er) und Neodym (Nd);

$Ln^2$ ausgewählt aus Gadolinium (Gd),

umfassend folgende Schritte:

- i) Bereitstellung mindestens eines Lanthanoidsalzes, ausgewählt aus Lanthanoidnitraten, Lanthanoidcarbonaten, Lanthanoidcarboxylaten, bevorzugt Lanthanoidacetate, Lanthanoidsulphate, Lanthanoidoxide, besonders bevorzugt $Pr_6O_{11}$ und/oder $Gd_2O_3$, wobei die Lanthanoidionen in den Lanthanoidoxiden oder Lanthanoidsalzen ausgewählt sind aus Praseodym, Gadolinium, Erbium, Neodym und für die Co-Dotierung mindestens zwei davon,

- ii) Bereitstellung eines Silikats, bevorzugt eines Silikatsalzes, besonders bevorzugt eines Alkalimetallsalzes des Silikats, oder eines Siliziumdioxids,

- iii) Bereitstellung mindestens eines Erdalkalimetallsalzes und mindestens eines Alkalimetallsalzes, bevorzugt eines Alkalimetallsilikats oder eines Alkalimetallcarbonats, ausgewählt aus einem Lithiumsalz oder einer Lithiumverbindung und optional ausgewählt aus einem Natriumsalz und Kaliumsalz, vorzugsweise das Salz des Lithiumsalzes, bevorzugt ein Lithiumsilikat, besonders bevorzugt ein Lithiumcarbonat, ein Calciumcarbonat und ein Natriumcarbonat,

- iv) Bereitstellung mindestens eines Flussmittels aus der Gruppe der Ammoniumhalogenide, vorzugsweise Ammoniumchlorid, Alkalimetallhalogenide, vorzugsweise Natriumchlorid, Natriumfluorid, Natriumbromid, Lithiumfluorid oder Lithiumchlorid, Erdalkalimetallhalogenide, vorzugsweise Calciumchlorid oder Calciumfluorid, und Lanthanoidhalogenide, vorzugsweise Praseodymfluorid oder Praseodymchlorid,

- a) Mischen der Komponente i), ii) iii) und iv) mittels Mahlens zur Erhaltung einer Mischung, oder

- b) Mischen der Komponente i), ii) und iii) und iv) in einem organischen polaren oder unpolaren Lösemittel, das kein protisches Lösemittel ist, mittels Mahlens zur Erhaltung einer Mischung;

- c) Vorkalzinierung der Mischung aus b) bei 600 bis 1000 °C, auch zur Entfernung der organischen Komponente, für mindestens 1 h, vorzugsweise mehr oder gleich 2 h, unter Luft-Atmosphäre zur Erhaltung einer vorkalzinierten Mischung, ggf. Abkühlung auf Raumtemperatur,

- d) Kalzinierung der Mischung aus a) oder der vorkalzinierten Mischung aus c) bei einer Temperatur von 600 bis < 1000 °C, bevorzugt bei 650 bis 900 °C, für mindestens 3 h, vorzugsweise für mindestens 12 h,

- e) Erhalten eines silikatbasierten Up-Conversion Leuchtstoffes der allgemeinen Formel (I), bevorzugt nach Abkühlen des Materials,

vorgeschlagen, wobei mindestens 3,5 Gew.-% Flussmittel, bezogen auf die Gesamtmenge der Edukte, eingesetzt werden.

Es konnte festgestellt werden, dass der Up-Conversion Leuchtstoff verbesserte Eigenschaften aufweist, wie z. B. die spezifische Oberfläche.

Ein weiterer Vorteil der Erfindung ist der Aspekt der Arbeitssicherheit. Durch eine Erhöhung der Flussmittelmenge wurde völlig unerwartet erreicht, dass der Einsatz von reduzierenden Gasen im Kalzinierungsschritt verzichtet werden konnte. Bei reduzierenden Gasen handelt es sich z. B. um CO-haltige Atmosphären oder um ein Formiergas, vorzugweise um Argon-Wasserstoff-Gemische oder Stickstoff-Wasserstoff-Gemische (97/3 und 95/5). Aus Gründen des Arbeits-, Gesundheits- und Umweltschutzes sind derartige reduzierende Gase ungünstig. Um den Arbeitsschutz aufgrund des Einsatzes dieser Gase für die Beschäftigten zu gewährleisten, ist es erforderlich, Vorkehrungen zu treffen beispielsweise durch einen apparativen Aufwand, was wiederum finanzieller Aufwand bedeutet.

Vorzugsweise kann das Verfahren unter Luft-Atmosphäre durchgeführt werden.

Bevorzugt beträgt bei dem erfindungsgemäßen Verfahren die Menge der Flussmittel nicht mehr als 50,0 Gew.-%, bevorzugt nicht mehr 10,0 Gew.-%, besonders bevorzugt nicht mehr als 4,0 Gew.-%, bezogen auf die Gesamtmenge der Edukte.

Es konnte gezeigt werden, dass die Partikelgrößenverteilung des erfindungsgemäßen gefluxten Leuchtstoffes einer Gauß'schen Verteilung ähnelt, was auf eine Homogenität der Partikelgröße hindeutet, sodass vorteilhafterweise deren Einarbeitung in einer Lackmatrix wesentlich leichter durchgeführt werden kann. Es wird angenommen, dass dadurch die Lackeigenschaften, wie etwa das Erscheinungsbild der Lackoberfläche, beispielsweise der Glanz, Haptik und Touch, verbessert wurden.

Auch konnte die Intensität der Emission der Up-Conversion Leuchtstoffe durch eine einfache technische Durchführung der Synthese erzielt werden.

Als bevorzugte Siliziumdioxide können die Produkte mit dem Markennamen Aerosil® 300, 200, OX50, 200V und 300V der Fa. Evonik verwendet werden.

Vorzugsweise wird als halogenhaltiges Flussmittel mindestens ein Stoff aus der Gruppe der Ammoniumhalogenide, Alkalimetallhalogenide, Erdalkalimetallhalogenide und Lanthanoidhalogenide eingesetzt. Halogenide dieser Gruppen haben überraschend gezeigt, dass damit hergestellte Up-Conversion Leuchtstoffe eine höhere Emission-Intensität aufweisen als mit anderen Flussmitteln.

Vorzugsweise handelt es sich bei den Halogeniden um Fluoride oder Chloride.

Bevorzugt handelt es sich bei den Alkalimetallen um Kalium, Natrium oder Lithium.

Bevorzugt handelt es sich bei dem Lanthanoid um Praseodym.

Bevorzugt handelt es sich bei den Erdalkalimetallen um Calcium oder Strontium.

Vorzugsweise wird der Leuchtstoff bei dem erfindungsgemäßen Verfahren mit Praseodym dotiert.

Bevorzugt wird der Leuchtstoff bei dem erfindungsgemäßen Verfahren mit Praseodym dotiert und mit Gadolinium co-dotiert.

Ein weiterer Gegenstand der Erfindung ist ein Up-Conversion Leuchtstoff der allgemeinen Formel (I)

$$A_{1-x-y-z}B^{*}_{y}B_{2}SiO_{4}{:}Ln^{1}_{x},Ln^{2}_{z}, \qquad\qquad I$$

mit

$x = 0,0001 - 0,0500$;
$z = 0,0000$ oder $z = 0,0001$ bis $0,3000$ mit der Maßgabe, dass gilt: $y = x + z$;
A ausgewählt aus der Gruppe bestehend aus Mg, Ca, Sr und Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
$Ln^{1}$ ausgewählt aus der Gruppe bestehend aus Praseodym (Pr), Erbium (Er) und Neodym (Nd);
$Ln^{2}$ ausgewählt aus Gadolinium (Gd), erhältlich nach dem erfindungsgemäßen Verfahren, wobei er eine spezifische Oberfläche, bestimmt durch Gasabsorption nach Brunauer, Emmett und Teller (BET) von 1 bis 500 $m^{2}$/g, bevorzugt 5 - 250 $m^{2}$/g, besonders bevorzugt 10 - 100 $m^{2}$/g, gemessen nach DIN 66131:1993-07 aufweist.

Vorzugsweise ist der Leuchtstoff ein kristallines Silikat oder aus kristallinen Silikaten, dotiert mit Lanthanoid-Ionen, umfassend mindestens einen Alkalimetallion und mindestens einen Erdalkalimetallion.

Bevorzugt wird der Leuchtstoff mit Praseodym dotiert und mit Gadolinium co-dotiert.

Es ist bevorzugt, dass der Leuchtstoff partiell oder vollständig kristallin ist. Vorzugsweise ist der Leuchtstoff also zumindest nicht vollständig amorph. Es ist daher bevorzugt, dass der Leuchtstoff keine amorph erstarrte Schmelze (Glas) ist. Der Leuchtstoff weist vorzugsweise einen kristallinen Anteil von > 50%, bevorzugt von > 70%, besonders bevorzugt > 85%, errechnet nach der Rechenformel (DOC = Degree of Crystallinity)

$$DOC = \frac{Kristalline\ Fl\ddot{a}che}{Kristalline\ Fl\ddot{a}che + Amorphe\ Fl\ddot{a}che}$$

mit Hilfe eines Röntgenpulverdiffraktogramms. Es wird auf die Beschreibung der Methode verwiesen.

Bevorzugt wird der Leuchtstoff ausgewählt aus Verbindungen der allgemeinen Formel (Ia)

$$A_{1-x-y-z}B^*_yB_2SiO_4:Pr_x,Gd_z, \qquad \textbf{(Ia)}$$

mit A ausgewählt aus der Gruppe bestehend aus Mg, Ca, Sr, Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
x = 0,0001 - 0,0500;
z = 0,0000 oder z = 0,0001 bis 0,3000 mit der Maßgabe, dass gilt: y = x + z.

B* dient dabei zum Ladungsausgleich der Praseodym- bzw. Gadolinium-Substitution..

A kann dabei für ein einziges Element aus der Gruppe bestehend aus Mg, Ca, Sr und Ba stehen, oder auch für eine Kombination von zwei oder mehr Elementen dieser Gruppe, also z.B. A = $(Mg_{a1}\ Ca_{a2}\ Sr_{a3}\ Ba_{a4})$ mit $0 \leq a1 \leq 1$, $0 \leq a2 \leq 1$, $0 \leq a3 \leq 1$ $0 \leq a4 \leq 1$ und mit der Maßgabe, dass gilt: a1+a2+a3+a4 = 1. A kann also z.B. für $(Ca_{0,9}Sr_{0,1})$ stehen.

Vorzugsweise wird der Leuchtstoff ausgewählt aus Verbindungen der allgemeinen Formel (II)

$$(Ca_{1-a}Sr_a)_{1-2b}Ln_bNa_bLi_2SiO_4 \qquad II$$

wobei gilt:

Ln ist ausgewählt aus der Gruppe bestehend aus Praseodym, Gadolinium, Erbium, Neodym, vorzugsweise Praseodym;
a = 0,0000 bis 1,0000, vorzugsweise 0,0000 bis 0,1000, insbesondere 0,0000;
b = 0,0001 bis 0,5000, vorzugsweise 0,0001 bis 0,1000, insbesondere 0,0050 bis 0,0500.

Ln kann dabei für ein einziges Element aus der Gruppe bestehend aus Praseodym, Gadolinium, Erbium und Neodym stehen, oder auch für eine Kombination von zwei Elementen dieser Gruppe, also z.B. Ln = $(Ln^1_x\ Ln^2_y)$, wobei $Ln^1$ und $Ln^2$ ausgewählt sind der Gruppe bestehend aus Praseodym, Gadolinium, Erbium und Neodym, und wobei x und y wie für Formel (I) und (Ia) definiert sind.

$Ln^1$ dient der Dotierung. Für die Dotierung wird vorzugsweise Praseodym verwendet. $Ln^2$ dient der optionalen Co-Dotierung. Für die optionale Co-Dotierung wird vorzugsweise Gadolinium verwendet. Vorzugsweise ist der Leuchtstoff nicht co-dotiert, d.h. Ln steht für vorzugsweise für ein einziges Element aus der Gruppe bestehend aus Praseodym, Gadolinium, Erbium und Neodym.

Es ist noch bevorzugter, dass der Leuchtstoff ausgewählt ist aus Verbindungen der allgemeinen Formel (IIa)

$$Ca_{1-2b}Pr_bNa_bLi_2SiO_4 \qquad \text{(IIa)}$$

mit b = 0,0001 bis 0,5000, vorzugsweise 0,0001 bis 0,1000, insbesondere 0,0050 bis 0,0500.

Es ist ganz besonders bevorzugt, dass der Leuchtstoff $Ca_{0,98}Pr_{0,01}Na_{0,01}LbSiO_4$ oder $Ca_{0,94}Pr_{0,03}Na_{0,03}Li_2SiO_4$ oder $Ca_{0,90}Pr_{0,05}Na_{0,05}Li_2SiO_4$ ist.

Bevorzugt weist der erfindungsgemäße Up-Conversion Leuchtstoff ein Halogen, entsprechend dem Halogenid des Flussmittels, auf.

Bevorzugt handelt es sich bei dem Leuchtstoff um einen, der elektromagnetische Strahlung mit geringerer Energie und höherer Wellenlänge im Bereich von 2000 nm bis 400 nm, insbesondere im Bereich von 800 nm bis 400 nm, in elektromagnetische Strahlung mit höherer Energie und kürzerer Wellenlänge im Bereich von 400 nm bis 100 nm, bevorzugt im Bereich von 300 nm bis 200 nm konvertiert. Es ist ferner bevorzugt, dass dabei die Intensität des Emissionsmaximums der elektromagnetischen Strahlung mit höherer Energie und kürzerer Wellenlänge eine Intensität von mindestens $1 \cdot 10^3$ counts/(mm²*s), bevorzugt höher als $1 \cdot 10^4$ counts/(mm²*s), besonders bevorzugt höher als $1 \cdot 10^5$ counts/(mm²*s). Zur Bestimmung dieser Kenngrößen wird die Emission bevorzugt mittels eines Lasers, insbesondere eines Lasers mit einer Leistung von 75 mW bei 445 nm und/oder einer Leistung von 150 mW bei 488 nm angeregt.

Vorzugsweise weist der Leuchtstoff gemäß Formel (II) XRPD Signale im Bereich von 23° 2θ bis 27° 2θ und von 34° 2θ bis 39,5° 2θ auf, wobei die Signale mittels der Bragg-Brentano Geometrie und der Cu-K$_\alpha$ Radiation bestimmt werden. Einzelheiten der Messmethode können aus der noch unveröffentlichten europäischen Patentanmeldungen EP 19202910.6 und PCT/EP2020/077798 entnommen werden.

Die PCT/EP2020/077798 widmen sich der Herstellung von Leuchtstoffen, insbesondere von Leuchtstoffen gemäß Formel (I), Formel (Ia) und Formel (II), ohne den Zusatz von Flussmitteln.

Weitere detaillierte Ausführungsformen des Verfahrens können aus der EP 19202910.6 und PCT/EP2020/077798 entnommen werden, wobei für das erfindungsgemäße Verfahren mindestens 3,5 Gew.-% Flussmittel, bezogen auf die Gesamtmenge der Edukte, zum Einsatz kommt.

Völlig überraschend konnte das bekannte Verfahren auf eine elegante Art und Weise modifiziert werden, was zudem zu optimierten Up-Conversion Leuchtstoffen mit außergewöhnlichen und unerwarteten Eigenschaften hinsichtlich der Partikelgrößenverteilung, Erhöhung der Emissionsintensität und spezifischen Oberfläche führt.

Es wird angenommen, dass die Zugabe von mehr als 3,5 Gew.-% Flussmittel zu einer homogeneren Kristallisations-/Schmelzprozess führt. In diesem könnten sich die Praseodym-Ionen homogener im Gitter verteilen und eine gleichmäßige Dotierung ermöglichen. Des Weiteren könnte der homogenere Schmelzprozess zu einer Sinterung der Partikeloberfläche und dadurch zu einer geringeren spezifischen Oberfläche der Up-Conversion Leuchtstoffe führen. Erfahrungsgemäß lassen sich Partikel mit geringerer spezifischer Oberfläche mit weniger Energieeintrag in die Beschichtungsmatrix einarbeiten.

Es hat sich überraschend herausgestellt, dass die erfindungsgemäßen Leuchtstoffe, hergestellt angelehnt an die Lehre der EP 19202910.6 und PCT/EP2020/077798 die erforderlichen Up-Conversion Eigenschaften aufweisen, die für die antimikrobielle Wirkung verantwortlich sind. D. h. diese Leuchtstoffe können elektromagnetische Strahlung mit Wellenlängen oberhalb der UV-Strahlung, insbesondere sichtbares Licht oder Infrarotlicht, in elektromagnetische Strahlung mit geringerer Wellenlänge konvertieren, und zwar in dem Bereich, bei dem z.B. die DNA bzw. RNA der Mikroorganismen zerstört bzw. mutiert werden kann. Demnach sind diese Leuchtstoffe für die erfindungsgemäße Zusammensetzung sehr gut geeignet.

Es sei hier zu erwähnen, dass es möglich ist, durch eine nachträgliche Vermahlung des Leuchtstoffes gemäß der Lehre der EP 19202910.6 und PCT/EP2020/077798 einerseits die Homogenität der Partikelgröße und andererseits die gewünschte Partikelgröße zu erreichen. Jedoch würde hierbei der Energieeintrag höher liegen und der Vermahlungsprozess länger dauern bedingt durch deren Inhomogenität und Partikelgrößenverteilung nach der Herstellung.

Auch Gegenstand der Erfindung ist die Verwendung der nach dem erfindungsgemäßen Verfahren hergestellten Leuchtstoffe zur Herstellung von Beschichtungen mit anti-mikrobieller Eigenschaft enthaltend

- mindestens ein filmbildendes Polymer,
- optional mindestens ein Additiv,
- optional mindestens einen Härter.

Die Auswahl filmbildender Polymere spielt hierbei eine wichtige Rolle. Grundsätzlich kommen alle aus dem Stand der Technik bekannten filmbildenden Polymere in Frage.

Vorzugsweise weist das filmbildende Polymer funktionelle Gruppen, vorzugsweise acide Wasserstoffe, auf, welche mit einem isocyanathaltigen Härter reaktiv sind und ggf. mit einem Katalysator katalysiert wird.

Vorteilhafterweise ist das filmbildende Polymer ausgewählt aus der Gruppe der hydroxyfunktionellen Acrylatpolymere, hydroxyfunktionellen Polyesterpolymere, und/oder hydroxyfunktionellen Polyetherpolymere, hydroxyfunktionellen Cellulosederivate, aminofunktionellen Asparticpolymer oder Polyesterpolymere, das mit einem isocyanathaltigen Härter reagiert.

Vorzugsweise ist das filmbildende Polymer resonanzarm.

Dem Fachmann sind die physikalischen Wechselwirkungen an der Oberfläche bekannt. Je nach Material und dessen Materialoberfläche treten bei Lichteinfall eine Vielzahl von Effekten an der Oberfläche auf. Das auftreffende Licht wird zum Teil absorbiert, ein Teil wird reflektiert und je nach Materialoberfläche auch gestreut. Licht kann auch erst absorbiert und dann wieder emittiert werden. Bei opaken, halbdurchlässigen oder lichtdurchlässigen Materialien kann das Licht auch durch den Körper durchdringen (Transmission). In einigen Fällen wird das Licht an der Oberfläche sogar polarisiert oder gebeugt. Manche Objekte können sogar Licht emittieren (beleuchtete Anzeigen, LED-Segmente, Bildschirme), in einer anderen Lichtfarbe fluoreszieren oder phosphoreszieren (nachleuchten).

Resonanzarm bedeutet im Sinne dieser Erfindung, dass das filmbildende Polymer geringe Absorption, Reflexion, Remission und Streuung im UV-Bereich aufweist. Dagegen soll vorzugsweise die Transmission ausgeprägt sein.

Es konnte nämlich überraschend festgestellt werden, dass die erfindungsgemäße filmbildende Polymere, die resonanzarm sind, eine verbesserte antimikrobielle Wirkung aufweisen, bedingt dadurch, dass mehr elektromagnetische Strahlung mit geringerer Energie und höherer Wellenlänge im Bereich von 2000 nm bis 400 nm, insbesondere im Bereich von 800 nm bis 400 nm, transmittiert wird und resultierend daraus in mehr elektromagnetische Strahlung mit höherer Energie und kürzerer Wellenlänge im Bereich von 400 nm bis 100 nm, bevorzugt im Bereich von 300 nm bis 200 nm, konvertiert werden kann.

Es wurde festgestellt, dass je höher die Transmission ist, desto höher ist auch die Emission, die für die antimikrobielle Wirkung ausschlaggebend ist.

Vorzugsweise liegt die Transmission des filmbildenden Polymers bei mindestens 75 %, bevorzugt mindestens 80 % und besonders bevorzugt mindestens 85 %, gemessen bei einer Wellenlänge von 260 nm.

Vorzugsweise liegt die Transmission des filmbildenden Polymers bei mindestens 75 %, bevorzugt mindestens 80 % und besonders bevorzugt mindestens 85 %, gemessen bei einer Wellenlänge von 500 nm.

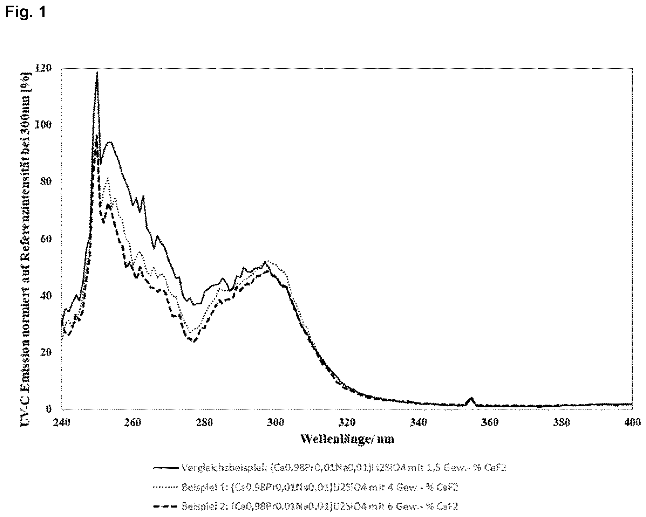

Zur Verdeutlichung sei hier angemerkt, dass die Transmission bei einer anderen Wellenlänge definiert werden kann, siehe Abbildung 1. Für die vorliegende Erfindung wurden die Wellenlängen 260 nm beispielhaft für die emittierte Wellenlänge und 500 nm beispielhaft für die Anregungswellenlänge gewählt, die zum einen für die Up-Conversion und zum anderen signifikant für die antimikrobielle Wirkung verantwortlich sind.

Bei einer Transmission von 100 % beispielsweise, gemessen bei einer Wellenlänge 260 nm, wird die gleiche Menge an Strahlung umgewandelt und emittiert, d. h. es gibt keine Verluste durch Absorption, Streuungen oder dergleichen. Bei einer Transmission von 80 %, gemessen bei einer Wellenlänge 260 nm, transmittiert 20 % nicht, vermutlich aufgrund von Absorption, Reflexion, Remission und/oder Streuung. Demzufolge kann nur 80 % der Strahlung der Wellenlänge 260 nm emittiert werden.

Diese bedeutende Erkenntnis ist wichtig bei der Auswahl der filmbildende Polymere. Polymeren, die z.B. 0 % Transmission aufweisen, eignen sich nicht für die erfindungsgemäße härtbare Zusammensetzung. Sie transmittieren keine elektromagnetische Strahlung mit geringerer Energie und höherer Wellenlänge und demzufolge können in der Zusammensetzung enthaltene Leuchtstoffe diese elektromagnetische Strahlung nicht in elektromagnetische Strahlung mit höherer Energie und kürzerer Wellenlänge umwandeln und emittieren, die für die antimikrobielle Wirkung erforderlich ist.

Vorzugsweise weist die erfindungsgemäße Zusammensetzung eine Transmission bei mindestens 75 %, bevorzugt bei mindestens 80 % und besonders bevorzugt bei mindestens 85 % gemessen bei 260 nm beträgt.

Vorzugsweise weist die erfindungsgemäße Zusammensetzung eine Transmission bei mindestens 75 %, bevorzugt bei mindestens 80 % und besonders bevorzugt bei mindestens 85 % gemessen bei 500 nm beträgt.

Die Transmissionskurven werden vorzugsweise mit einem "Specord 200 Plus" UV/VIS-Zweistrahlspektrometer der Firma Analytik Jena gemessen. Mit einem Holmiumoxidfilter erfolgt eine interne Wellenlängenkalibrierung. Die Proben werden mit monochromatisiertem Licht einer Deuterium- (UV-Bereich) oder einer Wolfram-Halogen-Lampe (sichtbarer Bereich) durchstrahlt. Die spektrale Bandbreite beträgt 1,4 nm. Das monochromatisierte Licht wird in einen Mess- und einen Referenzkanal aufgeteilt und ermöglicht das direkte Messen gegen eine Referenzprobe. Die durch die Probe transmittierende Strahlung wird von einer Photodiode detektiert und zu elektrischen Signalen verarbeitet.

Es ist vorstellbar, eine Zusammensetzung mit einer geringen Transmission von unter 70 % einzusetzen; sie wirken möglicherweise auch noch antimikrobiell, jedoch ist der Wirkungsgrad sehr moderat.

Vorzugsweise weisen die Leuchtstoffe eine durchschnittliche Partikelgröße von $d_{50}$ von 0,1 - 50 $\mu$m, bevorzugt $d_{50}$ = 0,1 - 25 $\mu$m, besonders bevorzugt $d_{50}$ = 0,1 - 5 $\mu$m, gemessen nach ISO 13320:2020 und USP 429, beispielsweise mit einem Gerät der Fa. Horiba, LA-950 Laser Particle Size Analyzer, auf.

Um die Leuchtstoffe in der erfindungsgemäßen Zusammensetzung gut einzubinden und/oder zu stabilisieren, können vorzugsweise verschiedene Additive zugesetzt werden.

Bevorzugt sind die Additive ausgewählt aus der Gruppe der Dispergiermittel, Rheologiehilfsmittel, Verlaufsmittel, Netzmittel, Entschäumer und UV-Stabilisierungsmittel.

Es wurde überraschend festgestellt, dass durch etwaigen Zusatz an Additiven in die erfindungsgemäße Zusammensetzung die Transmission reduziert wird.

Demnach weist die erfindungsgemäße Zusammensetzung in einer weiteren Ausführungsform, in der Additive eingesetzt werden, vorzugsweise eine Transmission bei mindestens 70 %, bevorzugt bei mindestens 75 % und besonders bevorzugt bei mindestens 80 % gemessen bei 260 nm beträgt.

Demnach weist die erfindungsgemäße Zusammensetzung in einer weiteren Ausführungsform, in der Additive eingesetzt werden, vorzugsweise eine Transmission bei mindestens 70 %, bevorzugt bei mindestens 75 % und besonders bevorzugt bei mindestens 80 % gemessen bei 500 nm beträgt.

Vorzugsweise weist die erfindungsgemäße Zusammensetzung einen Härter auf, der ausgewählt ist aus der Gruppe der aliphatischen oder cycloaliphatischen Isocyanate.

Beispiele für isocyanathaltige Härter sind monomere Isocyanate, polymere Isocyanate und Isocyanat-Prepolymere. Polyisocyanate werden gegenüber monomeren Isocyanaten aufgrund ihrer geringeren Toxizität bevorzugt.

Beispiele für Polyisocyanate sind Isocyanurate, Uretdione und Buirete basierend auf Diphenylmethan Diisocyanat (MDI), Toluol Diisocyanat (TDI), Hexamethylen Diisocyanate (HDI) and Isophoron Diisocyanat (IPDI). Beispiele für kommerziell erhältliche Produkte sind unter dem Markennamen DESMODUR@ von Covestro oder VESTANAT von der Evonik Industries. Bekannte Produkte sind DESMODUR@ N3400, DESMODUR@ N3300, DESMODUR@ N3600 DESMODUR@ N75, DESMODUR@ XP2580, DESMODUR@ Z4470, DESMODUR@ XP2565 and DESMODUR@ VL, von Covestro. Weitere Beispiele sind VESTANAT® HAT 2500 LV, VESTANAT® HB 2640 LV oder VESTANAT® T 1890E von der Evonik Industries. Beispiele für Isocyanat-Prepolymere sind DESMODUR@ E XP 2863, DESMODUR@ XP 2599 oder DESMODUR@ XP 2406 von Covestro. Weitere dem Fachmann bekannte Isocyanat-Prepolymere können eingesetzt werden.

Es ist vorstellbar, Katalysatoren zur Aushärtung einzusetzen. Folgende Katalysatoren ausgewählt aus organischen Sn(IV)-, Sn(II)-, Zn-, Bi-Verbindungen oder tertiären Aminen können eingesetzt werden.

Bevorzugt werden Katalysatoren ausgewählt aus der Gruppe der Organozinnkatalysatoren, Titanate oder Zirkonate, metallorganische Verbindungen des Aluminiums, Eisens, Calciums, Magnesiums, Zinks oder Bismuts, Lewis-Säuren oder organische Säuren/Basen, lineare oder zyklische Amidine, Guanidine oder Amine oder eine Mischung daraus eingesetzt.

Vorzugseise werden als Härtungs-Katalysatoren organische Zinnverbindungen, wie z.B. Dibutylzinndilaurat, Dibutylzinndiacetylacetonat, Dibutylzinndiacetat, Dibutylzinndioctoat, oder Dioctylzinndilaurat, Dioctylzinndiacetylacetonat, Dioctylzinndiketanoat, Dioctylstannoxan, Dioctylzinndicarboxylat, Dioctylzinnoxid, bevorzugt Dioctylzinndiacetylacetonat, Dioctylzinndilaurat, Dioctylzinndiketanoat, Dioctylstannoxan, Dioctylzinndicarboxylat, Dioctylzinnoxid, besonders bevorzugt Dioctylzinndiacetylacetonat und Dioctylzinndilaurat eingesetzt. Des Weiteren können auch Zinksalze, wie Zinkoctoat, Zinkacetylacetonat und Zink-2-ethylcaproat, oder Tetraalkylammoniumverbindungen, wie N,N,N-Trimethyl-N-2-hydroxypropyl-ammonium-hydroxid, N,N,N-Trimethyl-N-2-hydroxypropylammonium-2-ethylhexanoat oder Cholin-2-ethylhexanoat verwendet werden. Bevorzugt ist die Verwendung von Zinkoctoat (Zink-2-ethylhexanoat) und der Tetraalkylammoniumverbindungen, besonders bevorzugt diejenige von Zinkoctoat. Weiter bevorzugt sind Bismutkatalysatoren, z.B. TIB Kat (TIB Mannheim) oder Borchi®-Katalysatoren, Titanate, z.B. Titan(IV)isopropylat, Eisen(III)-Verbindungen, z.B. Eisen(III)-acetylacetonat, Aluminiumverbindungen, wie Aluminiumtriisopropylat, Aluminiumtri-secbutylat und andere Alkoholate sowie Aluminiumacetylacetonat, Calciumverbindungen, wie Calciumdinatriumethylendiamin-tetraacetat oder Calciumdiacetylacetonat, oder auch Amine, z.B. Triethylamin, Tributylamin, 1,4-Diazabicyclo[2,2,2]octan, 1,8-Diazabicyclo[5.4.0]undec-7-en, 1,5-Diazabicyclo[4.3.0]non-5-en, N,N-Bis-(N,N-dimethyl-2-aminoethyl)-methylamin, N,N-Dimethylcyclohexylamin, N,N-Dimethylphenylamin, N-Ethylmorpholin etc.. Auch organische oder anorganische Brönstedsäuren wie Essigsäure, Trifluoressigsäure, Methansulfonsäure, p-Toluolsulfonsäure oder Benzoylchlorid, Salzsäure, Phoshorsäure deren Mono- und/oder Diester, wie z.B. Butylphosphat, (Iso-)Propylphosphat, Dibutylphosphat etc., sind als Katalysatoren bevorzugt. Weiter bevorzugt sind Guanidin-Gruppen tragende organische und Silicium-organische Verbindungen. Selbstverständlich können auch Kombinationen mehrerer Katalysatoren eingesetzt werden. Darüber hinaus können auch photolatente Basen als Katalysatoren verwendet werden, wie sie in der WO 2005/100482 beschrieben sind.

Der Härtungskatalysator wird vorzugsweise in Mengen von 0,01 bis 5,0 Gew.-%, bevorzugt 0,05 bis 4,0 Gew.-% und besonders bevorzugt 0,1 bis 3 Gew.-% bezogen auf das Gesamtgewicht der härtbaren Zusammensetzung eingesetzt.

Bei filmbildenden Polymeren, die durch physikalische Trocknung aushärten, ist die Zugabe von reaktiven Härtern nicht erforderlich.

Die erfindungsgemäße Zusammensetzung können vorzugsweise in 1K-Beschichtungssysteme oder 2K- Beschichtungssysteme, in Melamin Einbrenn-Systeme, Raum- oder Hochtemperatursysteme eingesetzt werden.

Vorzugsweise weisen aus der erfindungsgemäßen Zusammensetzung hergestellte Beschichtungen eine antimikrobielle Wirkung gegen Bakterien, Hefen, Schimmelpilze, Algen, Parasiten und Viren auf.

Bevorzugt weisen die erfindungsgemäßen hergestellten Beschichtungen eine antimikrobielle Wirkung gegen

- Erreger nosokomialer Infektionen, vorzugsweise gegen *Enterococcus faecium, Staphylococcus aureus, Klebsiella pneumoniae, Acinetobacter baumannii, Pseudomonas aeruginosa, Escherichia coli, Enterobacter, Corynebacterium diphteria, Candida albicans,* Rotavirus, Bakteriophagen;
- Fakultativ pathogene Umweltorganismen, vorzugsweise gegen *Cryptosporidium parvum, Giardia lamblia,* Amöben (*Arcanthamoeba* spp., *Naegleria* spp.), *E. coli,* coliforme Bakterien, Fäkalstreptokokken, *Salmonella* spp., *Shigella* spp. *Leginonella* spec., *Pseudomonas aeruginosa, Mykobakteria* spp., enterale Viren (z.B. Polio- und Hepatitis-A Virus);
- Pathogene in Lebensmitteln, vorzugsweise gegen *Bacillus cereus, Campylobacter* spp., *Clostridium botulinum, Clostridium perfringens, Cronobacter* spp., *E. coli, Listeria monocytogenes, Salmonella* spp., *Staphylococcus aureus, Vibrio* spp., *Yersinia enterocolitica,* Bakteriophagen,

auf.

Es wurde festgestellt, dass die Einarbeitung der erfindungsgemäßen Up-Conversion Leuchtstoffe deutlich verbessert wurde.

Up-Conversion Leuchtstoffe und Leuchtstoffe werden als Synonyme verwendet.

Ein weiterer Gegenstand ist die Verwendung der Leuchtstoffe in Zusammensetzungen zur Herstellung von Dispersionen, Mahlgütern, Klebstoffen, Spachtelmassen, Putzen, Farben, Lacken oder Drucktinten, Inkjets, Anreibeharzen oder Pigmentkonzentraten.

Vorzugsweise ist die Verwendung der erfindungsgemäßen Zusammensetzung zur Herstellung von Beschichtungen mit anti-mikrobieller Eigenschaft.

Eine Beschichtung mit antimikrobieller Wirkung oder antimikrobieller Eigenschaft bedeutet hierbei, dass die Beschichtung eine antimikrobielle Oberfläche aufweist, die das Wachstum und die Vermehrung von Mikroorganismen begrenzt oder verhindert.

Es wurde erstaunlicherweise auch festgestellt, dass die erfindungsgemäßen Beschichtungen eine chemische und mechanische Beständigkeit aufweisen. Die chemische und mechanische Beständigkeit ist besonders bedeutsam, da antimikrobielle Beschichtungen häufig in Bereichen eingesetzt werden, die eine regelmäßige Desinfektion und weitere Hygienemaßnahmen erfordern.

Auch zur Erfindung gehört ein Verfahren zur Bildung einer anti-mikrobiellen Beschichtung auf einem Substrat, umfassend das Aufbringen einer härtbaren filmbildenden Zusammensetzung auf das Substrat, umfassend:

a. mindestens ein filmbildendes Polymer, das funktionelle Gruppen enthält, welche mit einem isocyanathaltigen Härter reaktiv sind, ggf. durch einen Katalysator katalysiert,
b. mindestens einen Leuchtstoff gemäß der Formel (II) und
c. einen Härter, enthaltend isocyanatfunktionelle Gruppen.

Vorzugsweise handelt es sich bei dem Substrat um Metall, mineralische Substrate (wie etwa Beton, Naturstein oder Glas), zellulosehaltige Untergründe, Holz sowie deren Hybride, formstabile Kunststoffe und/oder Duromere.

Unter dem Begriff "formstabile Kunststoffe" werden folgende, jedoch nicht abschließende, Polymere verstanden: Acrylnitril-Butadien-Styrol (ABS), Polyamide (PA), Polylactat (PLA), Polymethylmethacrylat (PMMA), Polycarbonat (PC), Polyethylenterephthalat (PET), Polystyrol (PS), Polyetheretherketon (PEEK), Polyvinylchlorid (PVC), Polypropylen (PP), Polyethylen (PE).

Bevorzugt kann auf das Substrat vor der Auftragung der härtbaren filmbildenden Zusammensetzung eine Grundierzusammensetzung aufgetragen werden.

Vorzugsweise wird die erfindungsgemäße härtbare Zusammensetzung zur Beschichtung von Substraten in Hygieneeinrichtungen, Krankenhäusern und in der Lebensmittelindustrie eingesetzt.

Es können alle Bereiche des öffentlichen Raums, wie z.B. Schule, Altenheim, Großküche oder Kindergarten in Frage kommen.

Eine weitere Erfindung ist der Gegenstand, der mindestens teilweise, vorzugsweise vollständig, mit der erfindungsgemäßen härtbaren Zusammensetzung beschichtet ist.

Es sei hier anzumerken, dass die Begrifflichkeiten "antimikrobieller Effekt", antimikrobielle Wirksamkeit", "antimikrobielle Wirkung" und "antimikrobielle Eigenschaft" als Synonyme verwendet werden.

Es sei hier anzumerken, dass der erfindungsgemäße Gegenstand vorzugsweise auch ohne Freisetzung eines antimikrobiellen Wirkstoffs eine antimikrobielle Wirkung aufweisen kann, wenn die Beschichtung spezielle Leuchtstoffe, wie in den Ansprüchen beschrieben, enthalten. Auf diese Weise wird ein physikalischer Weg beschritten, auf dem die Mikroorganismen dann abgetötet werden. Daher fallen solche Materialien nicht unter die Biozid-Verordnung (Verordnung (EU), Nr. 528/2012 des Europäischen Parlaments und des Rates vom 22. Mai 2012 in der aktuellen Fassung von 2019).

Nachfolgend werden Beispiele aufgeführt, die dem Fachmann allein zur Erläuterung dieser Erfindung dienen und stellen keinerlei Beschränkung des beanspruchten Gegenstandes dar.

## Methoden

**Partikelgrößenverteilung** nach ISO 13320:2020 und USP 429, mit einem Gerät der Fa. Horiba, LA-950 Laser Particle Size Analyzer

**Qualitative Elementanalyse** mittels EDX mit Tabletop 4000Plus von Hitachi, BSE-Detektor 15 kV, 1000fache Vergrößerung

**Pulver-XRD:** Die Röntgenpulverdiffraktogramme der Proben wurden mit einem Bruker D2 Phaser Pulver Diffraktometer, das in der Bragg-Brentano Geometrie arbeitet, aufgenommen, wobei Cu-K$_\alpha$ Strahlung und ein Linescan CCD Detektor verwendet wurde. Die Integrationszeit betrug 20 s, während die Schrittweite 0,017° 2θ betrug.

**Die Emissionsspektren** wurden mit Hilfe eines Edinburgh Instruments FLS920 Spektrometers, das mit einem 488 nm continuous-wave OBIS Laser der Firma Coherent sowie einem Peltier gekühlten (-20 °C) single-photon counting photomultiplier der Firma Hamamatsu (R2658P) ausgerüstet ist. Kantenfilters wurden zur Unterdrückung der Reflexe zweiter und höherer Ordnung, welche durch die Monochromatoren verursacht werden, verwendet.

**BET Oberflächen Messungen** nach ISO 9277, DIN 66131 mit dem Gerät der Firma Quantachrome, Nova 2000e.

Der Grad der **Kristallinität** (engl. Degree of Crystallinity = DOC) gewährt eine Aussage über das Verhältnis der kristallinen zu amorphen Fläche aller Komponenten in einem Pulver Diffraktogramm, wie oben unter Punkt **Pulver-XRD** beschrieben. Der Grad der Kristallinität wird berechnet aus der Gesamtfläche unter den kristallinen und amorphen Anteilen:

$$DOC = \frac{Kristalline \; Fläche}{Kristalline \; Fläche + Amorphe \; Fläche}$$

**Leuchtstoffe**

**Beispiel 1 Erfindungsgemäßer Leuchtstoff (Ca$_{0,98}$Pr$_{0,01}$Na$_{0,01}$)Li$_2$SiO$_4$ mit 4 Gew.- % CaF$_2$ als Flussmittel**

4,12 g CaCO$_3$, 3,11 g Li$_2$CO$_3$, 2,52 g SiO$_2$, 0,02 g Na$_2$CO$_3$, 0,07g Pr$_6$O$_{11}$, und 0,4 g CaF$_2$ wurden miteinander vermischt. Diese Mischung wurde bei 850 °C für 6 h in Luft kalziniert, was zum gewünschten Produkt führt. Der Leuchtstoff wurde für weitere Messungen entnommen.

BET: 3 m$^2$/g
Partikelgrößenverteilung:

D$_{10}$: 3 μm
D$_{50}$: 9 μm
D$_{90}$: 32 μm

Grad der Kristallinität: 89 %

**Beispiel 2 Erfindungsgemäßer Leuchtstoff (Ca$_{0,98}$Pr$_{0,01}$Na$_{0,01}$)Li$_2$SiO$_4$ mit 6 Gew.-% CaF$_2$ als Flussmittel**

4,12 g CaCO$_3$, 3,11 g Li$_2$CO$_3$, 2,52 g SiO$_2$, 0,02 g Na$_2$CO$_3$, 0,07g Pr$_6$O$_{11}$, und 0,62 g CaF$_2$ wurden miteinander vermischt. Diese Mischung wurde bei 850 °C für 6 h in Luft kalziniert, was zum gewünschten Produkt führt. Der Leuchtstoff wurde für weitere Messungen entnommen.

BET: 2 m$^2$/g
Partikelgrößenverteilung:

D$_{10}$: 3 μm
D$_{50}$: 10 μm
D$_{90}$: 60 μm

Grad der Kristallinität: 90 %

**Vergleichsbeispiel: Leuchtstoff (Ca$_{0,98}$Pr$_{0,01}$Na$_{0,01}$)Li$_2$SiO$_4$ mit 1,5 Gew.-% CaF$_2$ als Flussmittel**

4,12 g CaCO$_3$, 3,11 g Li$_2$CO$_3$, 2,52 g SiO$_2$, 0,02 g Na$_2$CO$_3$, 0,07g Pr$_6$O$_{11}$, und 0,15 g CaF$_2$ wurden miteinander vermischt. Diese Mischung wurde bei 850 °C für 6 h in Luft kalziniert, was zum gewünschten Produkt führt. Der Leuchtstoff wurde für weitere Messungen entnommen.

BET: 49 m$^2$/g
Partikelgrößenverteilung:

D10: 3 μm
D50: 12 μm
D90: 56 μm

Grad der Kristallinität: 93 %

Die Partikelgrößenverteilung der erfindungsgemäßen Leuchtstoffe (Beispiel 1 und 2) und das Vergleichsbeispiel weisen keine signifikante Veränderung auf. Durch die Zugabe von 4 Gew.-% bzw. 6 Gew.-% CaF$_2$ reduziert sich die spezifische Oberfläche (BET) der erfindungsgemäßen Leuchtstoffe (Beispiel 1 und 2) signifikant im Vergleich zum Leuchtstoff mit 1,5 Gew.-%. Eine Verringerung der BET Oberfläche, bei gleichzeitig stabiler Partikelgrößenverteilung, deutet auf eine Verringerung der Porosität hin. Der Grad der Kristallinität der Leuchtstoffe ändert sich nicht signifikant durch die Zugabe von erhöhten CaF$_2$ Beimischungen.

Alle Leuchtstoffe zeigten eine Up-Conversion Eigenschaft im Emissionsspektrum im UV-C Bereich und eine anti-mikrobielle Wirkung auf. Die Einarbeitung der erfindungsgemäßen Leuchtstoffe in die Lackmatrix war deutlich einfacher.

Fig, 1 zeigt ein Emissionsspektrum der Beispiele 1 und 2 sowie des Vergleichsbeispiels. Die Leuchtstoffe zeigten den gewünschten Wellenlängenbereich.

**Patentansprüche**

1. Verfahren zur Herstellung eines Up-Conversion Leuchtstoffes der allgemeinen Formel (I)

$$A_{1-x-y-z}B^*_yB_2SiO_4{:}Ln^1_x,Ln^2_z, \qquad I$$

mit

x = 0,0001 - 0,0500;
z = 0,0000 oder z = 0,0001 bis 0,3000 mit der Maßgabe, dass gilt: y = x + z;
A ausgewählt aus der Gruppe bestehend aus Mg, Ca, Sr und Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
Ln$^1$ ausgewählt aus der Gruppe bestehend aus Praseodym (Pr), Erbium (Er) und Neodym (Nd);
Ln$^2$ ausgewählt aus Gadolinium (Gd),

umfassend folgende Schritte:

- i) Bereitstellung mindestens eines Lanthanoidsalzes, ausgewählt aus Lanthanoidnitraten, Lanthanoidcarbonaten, Lanthanoidcarboxylaten, bevorzugt Lanthanoidacetate, Lanthanoidsulphate, Lanthanoidoxide, besonders bevorzugt Pr$_6$O$_{11}$ und/oder Gd$_2$O$_3$, wobei die Lanthanoidionen in den Lanthanoidoxiden oder Lanthanoidsalzen ausgewählt sind aus Praseodym, Gadolinium, Erbium, Neodym und für die Co-Dotierung mindestens zwei davon,
- ii) Bereitstellung eines Silikats, bevorzugt eines Silikatsalzes, besonders bevorzugt eines Alkalimetallsalzes des Silikats, oder eines Siliziumdioxids,
- iii) Bereitstellung mindestens eines Erdalkalimetallsalzes und mindestens eines Alkalimetallsalzes, bevorzugt eines Alkalimetallsilikats oder eines Alkalimetallcarbonats, ausgewählt aus einem Lithiumsalz oder einer Lithiumverbindung und optional ausgewählt aus einem Natriumsalz und Kaliumsalz, vorzugsweise das Salz des Lithiumsalzes, bevorzugt ein Lithiumsilikat, besonders bevorzugt ein Lithiumcarbonat, ein Calciumcarbonat und ein Natriumcarbonat,
- iv) Bereitstellung mindestens eines Flussmittels aus der Gruppe der Ammoniumhalogenide, vorzugsweise Ammoniumchlorid, Alkalimetallhalogenide, vorzugsweise Natriumchlorid, Natriumfluorid, Natriumbromid, Lithiumfluorid oder Lithiumchlorid, Erdalkalimetallhalogenide, vorzugsweise Calciumchlorid oder Calciumfluorid, und Lanthanoidhalogenide, vorzugsweise Praseodymfluorid oder Praseodymchlorid,
- a) Mischen der Komponente i), ii) iii) und iv) mittels Mahlens zur Erhaltung einer Mischung, oder

- b) Mischen der Komponente i), ii) und iii) und iv) in einem organischen polaren oder unpolaren Lösemittel, das kein protisches Lösemittel ist, mittels Mahlens zur Erhaltung einer Mischung;
- c) Vorkalzinierung der Mischung aus b) bei 600 bis 1000 °C, auch zur Entfernung der organischen Komponente, für mindestens 1 h, vorzugsweise mehr oder gleich 2 h, unter Luft-Atmosphäre zur Erhaltung einer vorkalzinierten Mischung, ggf. Abkühlung auf Raumtemperatur,
- d) Kalzinierung der Mischung aus a) oder der vorkalzinierten Mischung aus c) bei einer Temperatur von 600 bis < 1000 °C, bevorzugt bei 650 bis 900 °C, für mindestens 3 h, vorzugsweise für mindestens 12 h,
- e) Erhalten eines silikatbasierten Up-Conversion Leuchtstoffes der allgemeinen Formel (I), bevorzugt nach Abkühlen des Materials,

**dadurch gekennzeichnet, dass** mindestens 3,5 Gew.-% Flussmittel, bezogen auf die Gesamtmenge der Edukte, eingesetzt werden.

2. Verfahren nach Anspruch 1, **dadurch gekennzeichnet, dass** die Menge der Flussmittel nicht mehr als 50,0 Gew.-%, bevorzugt nicht mehr 10,0 Gew.-%, besonders bevorzugt nicht mehr als 4,0 Gew.-%, bezogen auf die Gesamtmenge der Edukte beträgt.

3. Verfahren nach einem der vorgenannten Ansprüche, **dadurch gekennzeichnet, dass** die Kalzinierung (Schritt d) unter Luft-Atmosphäre durchgeführt wird.

4. Verfahren nach einem der vorgenannten Ansprüche, **dadurch gekennzeichnet, dass** es sich bei den Lanthanoiden um Praseodym handelt.

5. Verfahren nach einem der vorgenannten Ansprüche, **dadurch gekennzeichnet, dass** es sich bei den Alkalimetallen um Natrium oder Lithium handelt.

6. Verfahren nach einem der vorgenannten Ansprüche, **dadurch gekennzeichnet, dass** es sich bei den Erdalkalimetallen um Calcium handelt.

7. Verfahren nach einem der vorgenannten Ansprüche, **dadurch gekennzeichnet, dass** der Leuchtstoff mit Praseodym dotiert ist.

8. Up-Conversion Leuchtstoff der allgemeinen Formel (I)

$$A_{1-x-y-z}B^*_yB_2SiO_4{:}Ln^1_x,Ln^2_z, \qquad \textbf{I}$$

mit

$x = 0{,}0001 - 0{,}0500$;
$z = 0{,}0000$ oder $z = 0{,}0001$ bis $0{,}3000$ mit der Maßgabe, dass gilt: $y = x + z$;
A ausgewählt aus der Gruppe bestehend aus Mg, Ca, Sr und Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
$Ln^1$ ausgewählt aus der Gruppe bestehend aus Praseodym (Pr), Erbium (Er) und Neodym (Nd);
$Ln^2$ ausgewählt aus Gadolinium (Gd), erhältlich nach einem Verfahren gemäß einem der vorgenannten Ansprüche, **dadurch gekennzeichnet, dass** er eine spezifische Oberfläche bestimmt durch Gasabsorption nach Brunauer, Emmett und Teller (BET) von 1 bis 500 $m^2/g$, bevorzugt 5 - 250 $m^2/g$, besonders bevorzugt 10 - 100 $m^2/g$, gemessen nach ISO 9277, DIN 66131 aufweist.

9. Leuchtstoff nach Anspruch 8, **dadurch gekennzeichnet, dass** der Leuchtstoff mit Praseodym dotiert und mit Gadolinium co-dotiert ist.

10. Leuchtstoff nach einem der Ansprüche 8 - 9, **dadurch gekennzeichnet, dass** der Leuchtstoff eine erstarrte Schmelze aus kristallinen Silikaten oder aus kristallinen Silikaten dotiert mit Lanthanoidilonen, umfassend mindestens einem Alkalimetallilon und mindestens einem Erdalkalimetallion, bevorzugt, dass die kristallinen Silikate mit Praseodym dotiert und optional co-dotiert mit Gadolinium sind.

11. Leuchtstoff nach einem der Ansprüche 8 - 10, **dadurch gekennzeichnet, dass** der Leuchtstoff zumindest partiell kristallin ist.

12. Leuchtstoff nach einem der Ansprüche 8 - 11, **dadurch gekennzeichnet, dass** der Leuchtstoff ausgewählt ist aus Verbindungen der allgemeinen Formel (Ia)

$$A_{1-x-y-z}B^*_y B_2 SiO_4 : Pr_x, Gd_z, \qquad Ia$$

mit A ausgewählt aus der Gruppe bestehend aus Mg, Ca, Sr, Ba;
B ausgewählt aus der Gruppe bestehend aus Li, Na, K, Rb und Cs;
B* ausgewählt aus der Gruppe bestehend aus Li, Na und K, wobei B gleich B* oder B ungleich B* ist, bevorzugt B und B* sind ungleich;
x = 0,0001 - 0,0500;
z = 0,0000 oder z = 0,0001 bis 0,3000 mit der Maßgabe, dass gilt: y = x + z.

13. Leuchtstoff nach einem der Ansprüche 8 - 11, **dadurch gekennzeichnet, dass** der Leuchtstoff ausgewählt ist aus Verbindungen der allgemeinen Formel (II)

$$(Ca_{1-a}Sr_a)_{1-2b}Ln_b Na_b Li_2 SiO_4 \qquad II$$

wobei gilt:

Ln ist ausgewählt aus der Gruppe bestehend aus Praseodym, Gadolinium, Erbium, Neodym, vorzugsweise Praseodym;
a = 0,0000 bis 1,0000, vorzugsweise 0,0000 bis 0,1000, insbesondere 0,0000;
b = 0,0001 bis 0,5000, vorzugsweise 0,0001 bis 0,1000, insbesondere 0,0050 bis 0,0500.

14. Leuchtstoff nach einem der Ansprüche 8 - 11, **dadurch gekennzeichnet, dass** der Leuchtstoff ausgewählt ist aus Verbindungen der allgemeinen Formel (IIa)

$$Ca_{1-2b}Pr_b Na_b Li_2 SiO_4 \qquad (IIa)$$

mit b = 0,0001 bis 1, vorzugsweise 0,0001 bis 0,1, insbesondere 0,005 bis 0,0500.

15. Leuchtstoff nach einem der Ansprüche 8 - 14, **dadurch gekennzeichnet, dass** der Leuchtstoff $Ca_{0,98}Pr_{0,01}Na_{0,01}Li_2SiO_4$ oder $Ca_{0,94}Pr_{0,03}Na_{0,03}Li_2SiO_4$ oder $Ca_{0,90}Pr_{0,05}Na_{0,05}Li_2SiO_4$ ist.

16. Leuchtstoff nach einem der Ansprüche 8 - 16, **dadurch gekennzeichnet, dass** der Leuchtstoff gemäße Formel (II) XRPD Signale im Bereich von 23° 2θ bis 27° 2θ und von 34° 2θ bis 39,5° 2θ aufweist.

17. Verwendung der Leuchtstoffe nach einem der Ansprüche 8 - 17 zur Herstellung von Beschichtungen mit anti-mikrobieller Eigenschaft enthaltend

• mindestens ein filmbildendes Polymer,
• optional mindestens ein Additiv,
• optional mindestens einen Härter

Fig. 1

Europäisches
Patentamt

European
Patent Office

Office européen
des brevets

**EUROPÄISCHER RECHERCHENBERICHT**

Nummer der Anmeldung

EP 23 16 1186

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | DE 102 38 399 A1 (PHILIPS INTELLECTUAL PROPERTY [DE]) 26. Februar 2004 (2004-02-26) * Absätze [0001], [0002], [0007]; Beispiele * ----- | 1-16 | INV. C09K11/77 |
| Y | EP 2 881 447 A1 (SUMITOMO METAL MINING CO [JP]; UNIV TOHOKU [JP]) 10. Juni 2015 (2015-06-10) * Absätze [0060] - [0063], [0087] * ----- | 1-17 | |
| Y | JIANG LINGLING ET AL: "Preparation and luminescence properties of yellow long-lasting phosphor Ca2ZnSi2O7:Eu2+, Dy3+", MATERIALS SCIENCE AND ENGINEERING: B, Bd. 178, Nr. 2, 9. November 2012 (2012-11-09), Seiten 123-126, XP028961114, ISSN: 0921-5107, DOI: 10.1016/J.MSEB.2012.10.022 * Introduction; Abbildungen 4,5,6 * ----- | 1-17 | RECHERCHIERTE SACHGEBIETE (IPC) C09K |
| Y | WO 2021/073915 A1 (EVONIK OPERATIONS GMBH [DE]) 22. April 2021 (2021-04-22) * Ansprüche 14, 19; Abbildungen 3-6; Beispiele 1-4 * ----- -/-- | 1-17 | |

Der vorliegende Recherchenbericht wurde für alle Patentansprüche erstellt

| Recherchenort | Abschlußdatum der Recherche | Prüfer |
|---|---|---|
| Den Haag | 10. Juli 2023 | Baldé, Kaisa |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes Dokument

EPO FORM 1503 03.82 (P04C03)

EUROPÄISCHER RECHERCHENBERICHT

Nummer der Anmeldung

EP 23 16 1186

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | JUNG KYEONG YOUL ET AL: "Effect of surface area and crystallite size on luminescent intensity of Y2O3:Eu phosphor prepared by spray pyrolysis", MATERIALS LETTERS, Bd. 59, Nr. 19, 20. April 2005 (2005-04-20), Seiten 2451-2456, XP029235606, ISSN: 0167-577X, DOI: 10.1016/J.MATLET.2005.03.017 * Abbildung 6 * ----- | 8-17 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 10. Juli 2023 | Baldé, Kaisa |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 23 16 1186

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-07-2023

| Im Recherchenbericht angeführtes Patentdokument | Datum der Veröffentlichung | Mitglied(er) der Patentfamilie | | Datum der Veröffentlichung |
|---|---|---|---|---|
| DE 10238399 A1 | 26-02-2004 | AT | 350430 T | 15-01-2007 |
| | | AU | 2003255922 A1 | 11-03-2004 |
| | | CN | 1678713 A | 05-10-2005 |
| | | DE | 10238399 A1 | 26-02-2004 |
| | | DE | 60310947 T2 | 30-08-2007 |
| | | EP | 1532224 A1 | 25-05-2005 |
| | | JP | 2005536843 A | 02-12-2005 |
| | | TW | 200413500 A | 01-08-2004 |
| | | US | 2006108910 A1 | 25-05-2006 |
| | | WO | 2004018589 A1 | 04-03-2004 |
| EP 2881447 A1 | 10-06-2015 | EP | 2881447 A1 | 10-06-2015 |
| | | JP | 5578739 B2 | 27-08-2014 |
| | | JP | 2014043539 A | 13-03-2014 |
| | | TW | 201404868 A | 01-02-2014 |
| | | US | 2015203749 A1 | 23-07-2015 |
| | | WO | 2014021006 A1 | 06-02-2014 |
| WO 2021073915 A1 | 22-04-2021 | CN | 114555756 A | 27-05-2022 |
| | | EP | 4045610 A1 | 24-08-2022 |
| | | JP | 2022551693 A | 13-12-2022 |
| | | TW | 202124668 A | 01-07-2021 |
| | | US | 2022403239 A1 | 22-12-2022 |
| | | WO | 2021073915 A1 | 22-04-2021 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019197076 A
- DE 102015102427
- US 20090130169 A1
- WO 2009064845 A2
- EP 2020077798 W

- EP 3929254 A
- EP 21167984
- EP 19202910
- WO 2005100482 A